# EUROPEAN PATENT SPECIFICATION

(11) **EP 0 644 192 B1**
(45) Date of publication and mention of the grant of the patent: **18.12.1996**
(21) Application number: 94306644.9
(22) Date of filing: 09.09.1994
(51) Int. Cl.: C07D 417/12

(54) **A process for making a benzothiadiazole derivative**
Verfahren zur Herstellung eines Benzothiadiazolderivats
Procédé pour la préparation d'un dérivé de la benzothiadiazole

(30) Priority: 09.09.1993 JP 247276/93
(43) Date of publication of application: 22.03.1995
(73) Proprietor: PERMACHEM ASIA, LTD., Chuo-ku Tokyo (JP)
(72) Inventor: Ohwaki, Keiji, Ogasa-gun, Shizuoka-ken (JP); Yokogoshi, Kiyonori, Yokohama-shi, Kanagawa-ken (JP); Niino, Hideki, Yokohama-shi, Kanagawa-ken (JP)
(74) Representative: Woods, Geoffrey Corlett

(56) References cited:
- AU-A- 505 664
- CH-A- 579 565
- CH-A- 599 207
- DD-A- 246 764
- US-A- 3 843 668

## Description

The present invention relates to a process for producing a compound of the general name Tizanidine, i.e., 5-chloro-4-[(2-imidazoline-2-yl)amino]-2,1,3-benzothiadiazole. Tizanidine, particularly Tizanidine hydrochloride, is a clinically important medicament as a tonus lenitive.

Swiss Patent No. 579565 and US-A-3,843,668 describe that 2,1,3-benzothiadiazole derivatives including Tizanidine can be produced by reaction of ethylenediamine with a synthetic intermediate of one of the following formulae: 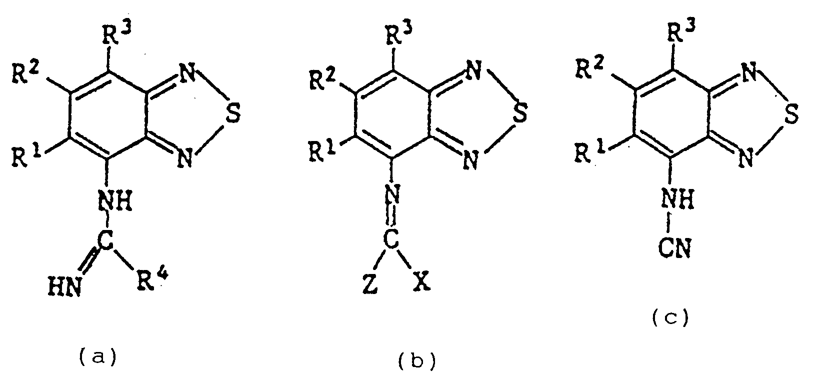 wherein R¹, R² and R³ independently represent hydrogen, halogen, an alkyl, alkoxy, nitro or hydroxy group, R⁴ represents a thio, amine or oxy group, X and Z independently represent a halogen atom, a thio, amine or oxy group.

However, the synthetic intermediates (a), (b) and (c) used in the prior process are difficult to obtain, and the use of highly poisonous thiophosgene is unavoidable in some cases. In addition, the process of making the compound of formula I by reaction of these synthetic intermediates with ethylenediamine is cumbersome in operation and is likely to cause side reactions, thus bringing about a tendency towards the lowering of yield.

Under these circumstances, it is still desired to provide a process as an industrial process of making the compound of formula I in high yield from inexpensive starting materials and in simple procedures. Hence, the object of the present invention is to provide a process for making the object compound Tizanidine of formula I, i.e., 5-chloro-4-[(2-imidazoline-2-yl)amino]-2,1,3-benzothiadiazole in high yield in simple reaction procedures.

In order to achieve the above object, the present inventors made extensive research on a process for making the compound of formula I in high yield in simple reaction procedures using starting materials or reaction agents available at lower costs. As a result, the present inventors found that the compound of formula I can be produced in extremely high yield by allowing a precursor of the above compounds (a), (b) and (c), i.e. 5-chloro-4-amino-2,1,3-benzothiadiazole, to react with a certain N-substituted-imidazolone.

According to the present invention, there is provided a process of producing 5-chloro-4-[(2-imidazoline-2-yl)amino]-2,1,3-benzothiadiazole of formula I: 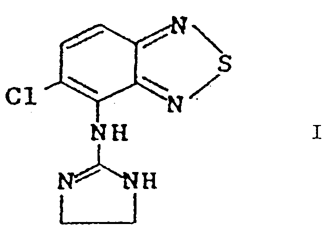 which process comprises:
(A) reacting the compound of formula (II): 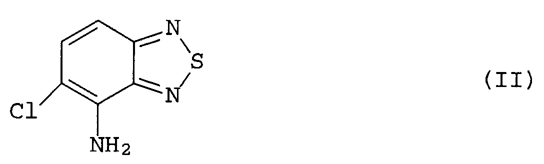 with a compound of formula (III): 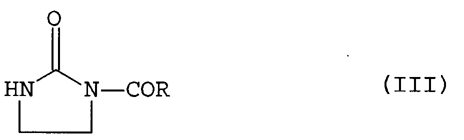 wherein R is a group selected from C₁-C₄ alkyl, C₃-C₇ cycloalkyl, phenyl, aryl-C₁-C₃ alkyl and C₁-C₄ alkyloxy, the alkyl or benzene skeleton radical in each case being optionally substituted by one or more halogen atoms, and the groups other than C₁-C₄ alkyloxy being optionally further substituted by a C₁-C₄ alkyloxy group, in the presence of a dehydrocondensation agent selected from sulfuric acid, phosphoric acid, sulfuryl chloride, phosphorus oxychloride, pyridine, dicyclohexyl carbodiimide, 1,8-diazabicyclo (5,4,0-) undecene-7 and 1,5-diazabicyclo (4,3,0-) nonene-5; and
(B) separating the compound of formula I from the resulting reaction mixture.

One of the starting materials in the present invention, the compound of formula II, is commercially available and is a per se known compound as described in the aforementioned Swiss Patent No. 579565. For a typical process of producing such an intermediate reference is made to e.g. V.G. Pesin and A.M. Khaletskii, Chem. Abst. Vol 52, p. 52 and ibid. 1952, p 106.

Another starting material, a compound of formula III, may also be a commercial product or can be readily obtained by the corresponding N-acylation or N-alkoxycarbonylation of imidazolone available as such at a lower cost. The group R in formula III can form, together with -CO-, an N-substituent group capable of elimination during the condensation reaction with the compound of formula II or followed by hydrolysis optionally carried out, as described below. Examples of the halogen substituent optionally on the alkyl radical or benzene skeleton radical are chlorine, bromine, iodine and fluorine. Examples of the C₁-C₄ alkyloxy substituent are methoxy, ethoxy, propoxy, isopropoxy, n-butoxy and tert-butoxy.

Examples of the C₁₋₄ alkyl group are methyl, ethyl, propyl, isopropyl, butyl, sec-butyl and tert-butyl; examples of the C₃₋₇ cycloalkyl group are cyclopropyl, cyclobutyl, cyclopentyl, cyclohexyl and cycloheptyl; and examples of the aryl-C₁₋₃ alkyl group are benzyl and phenethyl. Examples of the C₁₋₄ alkyloxy group R are methoxy, ethoxy, propoxy, iso-propoxy, butoxy, sec-butoxy and tert-butoxy.

The reaction in the above-described step (A) can, for example, be effected at a temperature of from 50°C to the reflux temperature of the reaction mixture in the presence of the dehydrocondensation agent. The reaction of step (A) is usually carried out for 2-40 hours, and if necessary under stirring. Although the coexistence of reaction solvent is usually not required since the dehydrocondensation agent can also function as solvent, there may coexist an inert solvent capable of raising the compatibility among the reaction agents and dehydrocondensation agent.

Examples of the inert solvent are alcohols such as methanol, ethanol, isopropyl alcohol and butanol; glycols such as ethylene glycol, diethylene glycol and MPG; ketones such as acetone and methyl ethyl ketone; ethers such as tetrahydrofuran, dioxane and diethyl ether; aromatic hydrocarbons such as toluene, xylene and benzene; and halogenated hydrocarbons such as dichloroethane and chloroform. Two or more types of the dehydrocondensation agents and inorganic solvents above enumerated can be used in the form of a mixture in such a range as not to adversely affect the condensation reaction of the step (A).

According to the reaction of the step (A), the compound of formula II and the compound of formula III can form the corresponding condensation product in one-pot with the substituent group -COR being eliminated therefrom, to yield the compound of formula I i.e. the object compound of the present invention. Depending on the type of the group -COR and the type of dehydrocondensation agent used, however, the procedure for condensation of the compounds of formulae II and III may be followed by the procedure of hydrolysis for promoting the elimination of the group -COR or for achieving the complete elimination of it.

This hydrolysis reaction is conducted preferably by treating with an aq. alkali solution the condensation product usually after separation from the reaction solvent and dehydrocondensation agent. For the preparation of such an alkali solution, it is convenient to employ, usually, alkali or alkaline earth metal hydroxides such as sodium hydroxide, potassium hydroxide and calcium hydroxide, or carbonates such as sodium carbonate and potassium carbonate. The aq. alkali solution containing such a hydroxide or carbonate may be an aq. hydroxide or carbonate itself or can be prepared by adding to such an aq. solution a water-miscible lower alcohol such as methanol, ethanol or isopropanol. The hydrolysis reaction can be effected usually for 1-10 hours with stirring at a temperature of from 50 °C to the reflux temperature of the solvent. For the separation of the resulting compound of formula I from the reaction mixture, the aq. alkali solution is concentrated and cooled by allowing it to stand, thus precipitating crystals which are then collected by filtration. Since the process of the invention can proceed considerably efficiently, it is possible to obtain the compound of formula I nearly quantitatively. The thus obtained crystals of formula I can be further purified by recrystallization from a suitable solvent such as methanol and chloroform.

According to the process of the invention, the object compound can be obtained in significantly high yield and in simple reaction procedures from the cheap and easily obtainable starting materials.

The following Examples illustrate the invention:

### Example 1

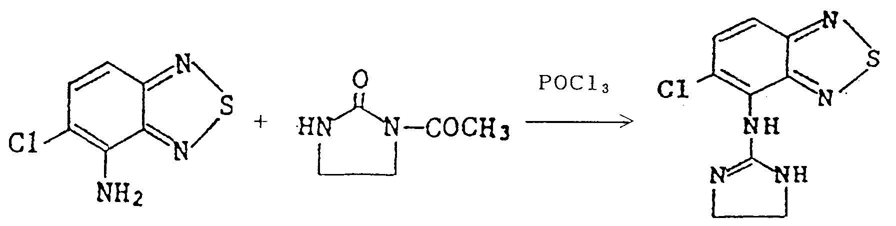

18.6 g of 5-chloro-4-amino-2,1,3-benzothiadiazole was added under stirring to 350 ml phosphorus oxychloride, followed by addition of 12.7 g of 1-acetyl-2-imidazolidinone, and the mixture was allowed to react at 50-60 °C for 30-40 hours.

After the conclusion of the reaction, the phosphorus oxychloride was distilled off under reduced pressure, and the residue was placed in ice-cold water and the pH of the solution was adjusted within an alkaline range with 1 N sodium hydroxide. The product was extracted several times with chloroform, and the chloroform layer was washed with water and dehydrated by addition of sodium sulfate anhydride, followed by removal of the chloroform.

The resulting crystals were dissolved in 150 ml of diluted sodium hydroxide/methanol (1 : 1) and stirred for 2-4 hours under heating, and then the methanol was distilled off. Subsequently, the reaction solution was cooled to precipitate crystals.

Filtration of the crystals gave 18.5 g of 5-chloro-4-[(2-imidazoline-2-yl)amino]-2,1,3-benzothiadiazole (yield: about 73 %). Melting point: 221.6 °C (recrystallized from methanol)

| Elementary analysis (determined with an elementary analysis apparatus type 240-C manufactured by Perkin-Elmer Co., Ltd. and calculated assuming that the molecular weight is 253.7) | | | | |
|---|---|---|---|---|
| | C | H | N | S |
| Theoretical values (%): | 42.61 | 3.18 | 27.60 | 12.64 |
| Found values (%): | 42.39 | 3.11 | 27.72 | 12.98 |

### Example 2

To 400 ml dry toluene were added 28 g of 5-chloro-4-amino-2,1,3-benzothiadiazole, 28.5 g of 1-benzoyl-2-imidazolidinone and 31 g of dicyclohexyl carbodiimide (DCC), and the mixture was allowed to react under reflux with heating.

After the consumption of the DCC was confirmed by TLC, the reaction was terminated, the reaction solution was cooled to room temperature and then filtered, and the toluene was distilled off.

The resulting crystals were dissolved in 300 ml of diluted sodium hydroxide/methanol (1 : 1), and the subsequent procedure was carried out in same manner as in Example 1 and the resulting crystals were converted into the corresponding hydrochloride salt in a usual manner to give 37 g of 5-chloro-4-[(2-imidazoline-2-yl)amino]-2,1,3-benzothiadiazole hydrochloride (yield : about 85 %).
Melting point: 290.2 °C (decomp.) (recrystallized from ethanol)

## Claims

1. A process for producing 5-chloro-4-[(2-imidazoline-2-yl) amino]-2,1,3-benzothiadiazole of formula (I): 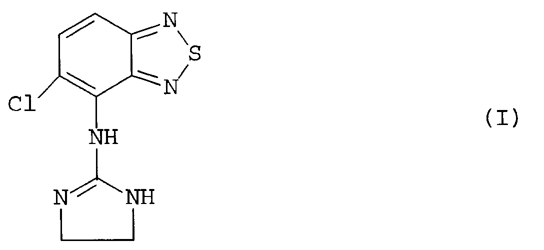 which process comprises:
(A) reacting the compound of formula (II): 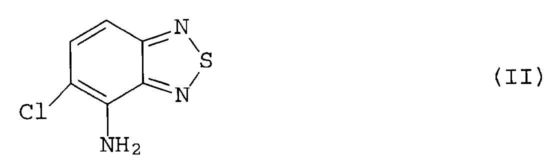 with a compound of formula (III); 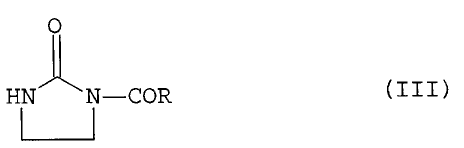 wherein R is a group selected from C₁-C₄ alkyl, C₃-C₇ cycloalkyl, phenyl, aryl-C₁-C₃ alkyl and C₁-C₄ alkyloxy, the alkyl or benzene skeleton radical in each case being optionally substituted by one or more halogen atoms, and the groups other than C₁-C₄ alkyloxy being optionally further substituted by a C₁-C₄ alkyloxy group, in the presence of a dehydrocondensation agent selected from sulfuric acid, phosphoric acid, sulfuryl chloride, phosphorus oxychloride, pyridine, dicyclohexyl carbodiimide, 1,8-diazabicyclo (5,4,0-) undecene-7 and 1,5-diazabicyclo (4,3,0-) nonene-5; and
(B) separating the compound of formula I from the resulting reaction mixture.

2. A process according to claim 1 in which step (A) is carried out in the presence of an inert solvent.

3. A process according to claim 1 or 2, which further comprises subjecting the reaction mixture resulting from step (A) to a hydrolysis reaction.

4. A process according to claim 3 wherein the hydrolysis reaction is carried out by treating the reaction mixture with an aqueous alkali solution.

5. A process according to claim 3 or 4, in which the hydrolysis reaction is carried out at a temperature from 50°C to the reflux temperature of the solvent.

6. A process according to claim 3, 4 or 5 in which the hydrolysis reaction is carried out for from 1 to 10 hours.

7. A process according to any one of the preceding claims in which step (A) is carried out at the reflux temperature of the reaction mixture.

8. A process according to any one of the preceding claims, in which step (A) is carried out for from 2 to 40 hours.

## Patentansprüche

1. Verfahren zur Herstellung von 5-Chlor-4-[(2-imidazolin-2-yl)amino]-2,1,3-benzothiadiazol der Formel (I): 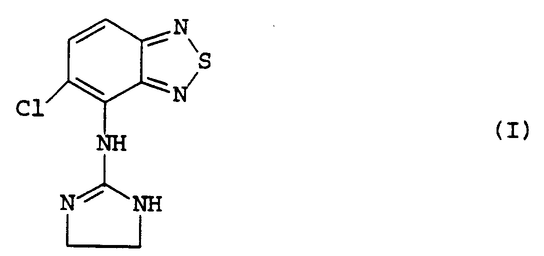 welches Verfahren umfaßt:
(A) Umsetzen der Verbindung der Formel (II): 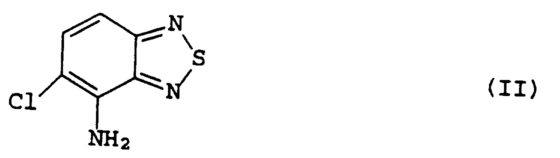 mit einer Verbindung der Formel (III): 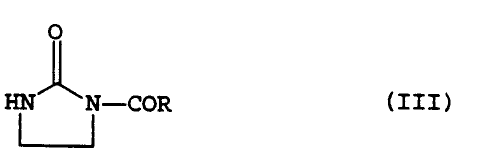 worin R eine Gruppe ist, gewählt aus C₁-C₄-Alkyl, C₃-C₇-Cycloalkyl, Phenyl, Aryl-C₁-C₃-alkyl und C₁-C₄-Alkyloxy, wobei das Alkyl- oder Benzol-Skelett-Radikal in jedem Fall wahlweise durch ein oder mehrere Halogenatome substitutiert ist, und die anderen Gruppen als C₁-C₄-Alkyloxy wahlweise weiter substitutert sind durch eine C₁-C₄-Alkyloxy-Gruppe, in Gegenwart eines Dehydro-Kondensationsmittels, gewählt aus Schwefelsäure, Phosphorsäure, Sulfurylchlorid, Phosphoroxidchlorid, Pyridin, Dicyclohexylcarbodiimid, 1,8-Diazabicyclo-(5,4,0)-undecen-7 und 1,5-diazabicyclo-(4,3,0)-nonen-5; und
(B) Trennen der Verbindung der Formel I vom resultierenden Reaktionsgemisch.

2. Verfahren nach Anspruch 1, bei welchem Schritt (A) in Gegenwart eines inerten Lösungsmittels durchgeführt wird.

3. Verfahren nach Anspruch 1 oder 2, welches ferner das Unterziehen des aus Schritt (A) resultierenden Reaktionsgemischs einer Hydrolyse-Reaktion umfaßt.

4. Verfahren nach Anspruch 3, worin die Hydrolyse-Reaktion durch Behandeln des Reaktionsgemischs mit einer wässrigen Alkalilösung vorgenommen wird.

5. Verfahren nach Anspruch 3 oder 4, bei welchem die Hydrolyse-Reaktion bei einer Temperatur von 50°C bis zur Rücklauftemperatur des Lösungsmittels vorgenommen wird.

6. Verfahren nach Anspruch 3, 4 oder 5, bei welchem die Hydrolyse-Reaktion über 1 bis 10 Stunden hinweg vorgenommen wird.

7. Verfahren nach einem der vorangegangenen Ansprüche, bei welchem Schritt (A) bei der Rücklauftemperatur des Reaktionsgemischs durchgeführt wird.

8. Verfahren nach einem der vorangegangenen Ansprüche, bei welchem Schritt (A) über 2 bis 40 Stunden hinweg durchgeführt wird.

## Revendications

1. Procédé de préparation du 5-chloro-4-[(2-imidazoline-2-yl)amino]-2,1,3-benzothiadiazole, de formule (I) : 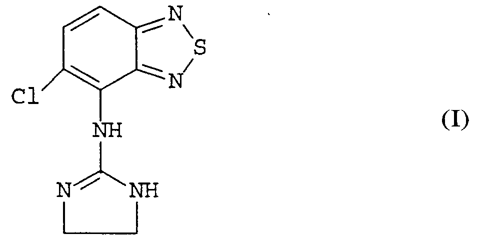 lequel procédé comporte
A) le fait de faire réagir le composé de formule (II) : 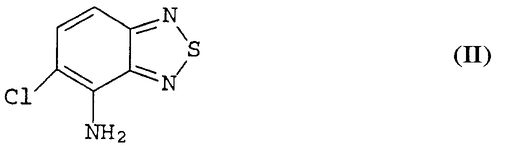 avec un composé de formule (III) : 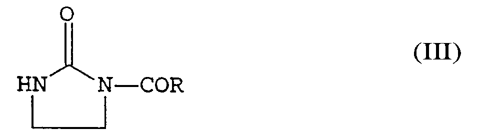 dans laquelle R représente un groupe choisi parmi les groupes alkyle en C₁₋₄, cycloalkyle en C₃₋₇, phényle, aryle-(alkyle en C₁₋₃) et alcoxy en C₁₋₄, dont le fragment alkyle ou aryle porte éventuellement comme substituants, dans chaque cas, un ou plusieurs atomes d'halogène, les groupes autres que les groupes alcoxy en C₁₋₄ portant en outre, éventuellement, un substituant alcoxy en C₁₋₄,
en présence d'un agent de condensation avec déshydratation, choisi parmi de l'acide sulfurique, de l'acide phosphorique, du chlorure de sulfuryle, de l'oxychlorure de phosphore, de la pyridine, du dicyclohexylcarbodiimide, du 1,8-diazabicyclo[5,4,0]undéc-7-ène et du 1,5-diazabicyclo[4,3,0]non-5-ène ;
et B) le fait de séparer le composé de formule (I) du mélange réactionnel résultant.

2. Procédé conforme à la revendication 1, dans lequel on effectue l'étape (A) en présence d'un solvant inerte.

3. Procédé conforme à la revendication 1 ou 2, qui comporte en outre le fait de soumettre à une réaction d'hydrolyse le mélange réactionnel issu de l'étape (A).

4. Procédé conforme à la revendication 3, dans lequel on effectue la réaction d'hydrolyse en traitant le mélange réactionnel avec une solution aqueuse alcaline.

5. Procédé conforme à la revendication 3 ou 4, dans lequel la réaction d'hydrolyse est effectuée à une température située entre 50°C et la température de reflux du solvant.

6. Procédé conforme à la revendication 3, 4 ou 5, dans lequel la réaction d'hydrolyse dure de 1 à 10 heures.

7. Procédé conforme à l'une des revendications précédentes, dans lequel l'étape (A) est effectuée à la température de reflux du mélange réactionnel.

8. Procédé conforme à l'une des revendications précédentes, dans lequel l'étape (A) dure de 2 à 40 heures.
